(19) Europäisches Patentamt
European Patent Office
Office européen des brevets

(11) **EP 4 569 523 B1**

(12) **EUROPEAN PATENT SPECIFICATION**

(45) Date of publication and mention
of the grant of the patent:
**22.07.2026  Bulletin 2026/30**

(21) Application number: **23762592.6**

(22) Date of filing: **09.08.2023**

(51) International Patent Classification (IPC):
*H01B 3/30* (2006.01)    *H01B 1/02* (2006.01)
*A61L 31/02* (2006.01)    *A61L 31/06* (2006.01)
*A61N 1/05* (2006.01)

(52) Cooperative Patent Classification (CPC):
(C-Sets available)
**H01B 3/306; A61L 31/022; A61L 31/10;
A61N 1/057**                              (Cont.)

(86) International application number:
**PCT/US2023/029825**

(87) International publication number:
**WO 2024/035769 (15.02.2024 Gazette 2024/07)**

(54) **COMPOSITE WIRE WITH COATING**

VERBUNDDRAHT MIT BESCHICHTUNG

FIL COMPOSITE AVEC REVÊTEMENT

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **10.08.2022   US 202263396712 P**

(43) Date of publication of application:
**18.06.2025   Bulletin 2025/25**

(73) Proprietor: **Fort Wayne Metals Research
Products, LLC
Fort Wayne, IN 46899 (US)**

(72) Inventors:
• **SCHAFFER, Jeremy E.
Fort Wayne, Indiana 46845 (US)**
• **GRIEBEL, Adam J.
Fort Wayne, Indiana 46816 (US)**

(74) Representative: **Stepney, Gregory John
Withers & Rogers LLP
2 London Bridge
London SE1 9RA (GB)**

(56) References cited:
**EP-A1- 3 415 649     US-A1- 2007 106 359
US-B2- 8 121 687**

(52) Cooperative Patent Classification (CPC): (Cont.)

C-Sets
**A61L 31/10, C08L 79/08**

**EP 4 569 523 B1**

**Description**

BACKGROUND

1. Technical Field

The present disclosure relates to composite wires and, in particular, to fatigue-resistant composite wires incorporating shape set NiTi with a polyimide coating.

2. Description of the Related Art

Since the first cardiac pacemaker implantation in 1960, these lifesaving devices have improved dramatically in capability, longevity, and reliability. The devices generally comprise two primary components: a pulse generator and one or more leads. Pulse generators comprise a battery and small computer in a hermetically sealed housing; their size often necessitates implantation some distance from the heart. The leads, complex assemblies of highly engineered wires and polymers, are used to transmit electrical signals to and from the generator and the heart tissue. Improvements in generator and lead technology combined with widespread adoption of cardiac pacemakers led to the commercialization of additional device types including implantable cardioverter defibrillators (ICD's), deep brain stimulators (DBS) and spinal cord as well as peripheral nerve stimulators. As these leads often need to function continuously for decades and can see hundreds of millions and even billions of cycles of flexural motion, fatigue resistance is a key design requirement.

Lead fatigue resistance is a function of construct geometry, material selection, and wire processing. Strain fatigue may be a greater concern compared to stress fatigue, as leads are generally not mechanically functional, and so simply need to move with the surrounding tissue without sustaining damage. Geometric configuration may guard against fatigue damage where incorporation of fine wires into strand, cable, or coil configurations reduces the maximum strain experienced by any individual wire. Proper wire alloy selection may also be used to mitigate stress fatigue.

Some of the earliest pacing leads were produced with Drawn Brazed Strand (DBS®) composite wires containing 316L stainless steel wires disposed around and brazed to a silver core [4]. In the 1980's, a new type of composite wire known as a DFT® (drawn filled tube) wire was introduced by Fort Wayne Metals of Fort Wayne, Indiana, with a MP35N® shell around a silver core. 35N LT® wire, with low titanium content, was later introduced to reduce titanium-nitride inclusions and substantially improved the fatigue resistance of the wire-based pacing leads. 35N LT and 35NLT®-DFT®-Ag wire (designating a shell of 35N LT and a core of Ag) are now the dominant materials used in biostimulation leads today and have a proven record of performance. A specially processed variant of 35N LT with a nanocrystalline microstructure (NDR® wire) and subsequent enhanced fatigue resistance has been more recently introduced and found utility in especially demanding lead applications. Patent Document US8121687B2 also discloses composite wires having a good fatigue resistance and being suitable to be employed in a medical device.

While the biostimulation leads in use today are well-served by 35N LT, materials with even further improvements in fatigue resistance would extend minimum service lifetimes and enable new indications and designs.

SUMMARY

A composite wire suitable for use as a pacing or biostimulation lead has improved durability when compared to currently available materials. Specifically, a lead includes a superelastic wire including, e.g., nitinol. The wire is coated with polyimide and subsequently wound into a strand, cable, coil, or helically stranded tube. This wound structure is thermomechanically treated to shape-set the wire construct and retain a desired wound and shaped configuration, without adversely affecting the integrity of the polyimide coating.

In one form thereof, the present disclosure provides a composite wire including a shell made of superelastic or shape memory alloy and defining a hollow cavity having an inner diameter, a core made of a conductive metal material and received within the hollow cavity and defining an outer diameter equal to the inner diameter, and a polymer coating disposed over the outer diameter of the shell. The composite wire exhibits a fatigue endurance surviving of at least $10^8$ cycles at 0.5% alternating strain.

In another form thereof, the present disclosure provides a coiled or wound wire including a shell made of nickel-titanium alloy and defining a hollow cavity having an inner diameter, a core made of a conductive metal material and received within the hollow cavity and defining an outer diameter equal to the inner diameter, and a polymer coating disposed over the outer diameter of the shell. At least the shell and the core are coiled or wound into a helical shape and configured to hold the helical shape without external forcing.

In yet another form thereof, the present disclosure provides a method of making a wire including inserting a conductive core into a hollow cavity of a shell made of nickel-titanium alloy to create a composite wire in which the core defines an outer diameter equal to an inner diameter of the hollow cavity, coating the composite wire with a polymer; and, after the step of coating the composite wire, shape setting the composite wire.

In yet another form thereof, the present disclosure provides a coiled or wound composite wire including a shell made of a nickel-titanium alloy, and defining a hollow cavity having an inner diameter, a core made of a conductive metal material, received within the hollow cavity, and defining an outer diameter equal to the inner diameter; and a polyimide coating disposed over the outer diameter of the shell, the polyimide coating having a thermal degradation, as measured by thermogravimetric analysis, of less than 10% at 500°C for 10 minutes or less in an inert environment. In this case, the composite wire exhibits a fatigue endurance surviving at least $10^8$ cycles at 0.5% alternating strain.

In yet another form thereof, the present disclosure provides a multifilament cable construct including a plurality of wires, each of the wires including a shell made of nickel-titanium alloy and defining a hollow cavity having an inner diameter, and a core made of a conductive metal material and received within the hollow cavity and defining an outer diameter equal to the inner diameter. In this case, he plurality of wires are configured into a helical shape which holds the helical shape without external forcing. The multifilament cable also includes a polyimide coating surrounding the plurality of coiled or wound wires where the polyimide coating has a thermal degradation, as measured by thermogravimetric analysis, of less than 10% at 500°C for not more than 10 minutes in an inert environment.

BRIEF DESCRIPTION OF THE DRAWINGS

The above mentioned and other features of this invention, and the manner of attaining them, will become more apparent and the invention itself will be better understood by reference to the following description of embodiments of the invention taken in conjunction with the accompanying drawings, where:

Fig. 1 is a schematic cross-section of a composite wire made in accordance with the present disclosure, including a nitinol shell or core;

Fig. 2 is a cross-section, elevation view of the composite wire of Fig. 1 further including a polyimide coating;

Fig. 3 is a graph showing rotating beam fatigue (R = -1) test data for Ø 0.18 mm wires of spring temper MP35N, 35N LT, 35N LT-NDR (nanocrystalline), and superelastic nitinol (Ni50.8Ti49.2) wire tested in RO water at 60 Hz and 310K;

Fig. 4 is a photograph of a 0.005" diameter NiTi-DFT-41%Ag wire coated with polyimide to .006" diameter, made into a coil of wire after heat treating in accordance with the present disclosure;

Fig. 5(a) is an electron SEM image of a bifilar coil made in accordance with the present disclosure, shown as a coiled finished construct;

Fig. 5(b) is an electron SEM image of a cross-section image of one of the wire elements of the bifilar coil of Fig. 5(a);

Fig. 5(c) is an electron SEM image of serial wire elements sectioned from the coil of Fig. 5(a) after polyimide coating, stranding and shape set heat treatment;

Fig. 6 is an electron SEM image of Cross-sections of a reference 35NLT-DFT-28Ag cable, shown at left, and a NiTi-DFT-30Ag cable made in accordance with the present disclosure, shown at right;

Fig.7(a) is a photograph of a portion of a coil tension-tension fatigue testing system, illustrating a 3.5 mm length bifilar coil specimen ready for loading to rigid grips and electrical continuity test unit;

Fig.7(b) is a photograph of a portion of the coil tension-tension fatigue testing system of Fig. 7(a), illustrating rigid steel grips clamped with electrical lead connections;

Fig.7(c) is a photograph of a portion of the coil tension-tension fatigue testing system of Fig. 7(a), illustrating the distilled water immersed 12 specimen fatigue system loaded with 6 fatigue specimens ready for cycling at 35 Hz and T = 310K ($\pm$ 2);

Fig. 8(a) is a tensile stress-strain curve graph for monofilament coated wires made of reference material and material in accordance with the present disclosure;

Fig. 8(b) is a tensile stress-strain curve graph for multifilament coated wires made of reference material and material in accordance with the present disclosure;

Fig. 8(c) is another tensile stress-strain curve graph for monofilament and multifilament coated wires made of material in accordance with the present disclosure;

Fig. 9 is a graph illustrating probability of fracture (N = 10) at less than $10^8$ cycles in rotating beam fatigue testing of 0.1 mm monofilament wires made of reference material and material in accordance with the present disclosure;

Fig. 10(a) is a photograph of a fixtured bifilar coil sample in situ to a tension-tension fatigue test device showing a clamped sample at 3.5 mm gage length;

Fig. 10(b) is a photograph of the clamped sample of Fig. 10(a) after extension to 200% macro structural strain (10.5 mm) mean condition;

Fig. 10(c) is a photograph of another fixtured bifilar 35NLT-DFT-28Ag coil sample in situ to a tension-tension fatigue test device showing fracture after 15% structural strain cycling to a total of 12 million cycles;

Fig. 10(d) is a photograph of a fixtured bifilar coil sample in situ to a tension-tension fatigue test device showing non-fracture in bifilar NiTi-DFT-30Ag made in accordance with the present disclosure, after 21.4% structural strain cycling;

Fig. 11 is a graph showing empirical probability of fracture at less than $10^7$ cycles for bifilar 35NLT-DFT-28Ag conductors at curve (a) and NiTi-DFT-30Ag conductors at curve (b);

Fig. 12 is a schematic view illustrating an exemplary process of forming composite wire using a lubricated drawing die;

Fig. 13 is an elevation view of a wire in accordance with the present disclosure, before a final cold working process; and

Fig. 14 is an elevation view of the wire of Fig. 16, after the final cold working process.

Corresponding reference characters indicate corresponding parts throughout the several views. Unless stated otherwise the drawings are to scale and proportional.

DETAILED DESCRIPTION

The embodiments disclosed below are chosen and described so that others skilled in the art may utilize their teachings.

As used herein, "wire" or "wire product" encompasses continuous wire and wire products which may be continuously produced and wound onto a spool for later dispensation and use, such as wire having a round cross section and wire having a non-round cross section, including flat wire or ribbon. "Wire" or "wire product" also encompasses other wire-based products such as strands, cables, coil, and tubing, which may be produced at a particular length depending on a particular application. Although round cross-sectional wire forms are shown in the Figures of the present application and described further below, non-round wire forms may also be produced in accordance with the present disclosure. Exemplary non-round forms include polygonal cross-sectional shapes such as rectangular cross-sectional shapes.

"Fine wire" refers to a wire having an outer diameter of less than 1 mm. "Ultrafine wire" refers to a wire having an outer diameter of 50 $\mu$m or less.

"Fatigue strength" refers to the load level at which the material meets or exceeds a given number of load cycles to failure. Herein, the load level is given as alternating strain, as is standard for displacement or strain-controlled fatigue testing, whereby terms are in agreement with those given in ASTM E606.

For purposes of the specific materials discussed below, fatigue strength is assessed via rotary beam fatigue testing. Such testing may be performed using a wire sample that is cut to an appropriate length (e.g., approximately 118 mm for a 0.33 mm diameter wire), then secured at its axial ends to rotatable jaws. The free portion of the wire between the jaws is bent to introduce a desired tensile strain at the "peak" or outermost portion of the bend. Directly opposite this peak of the bend, the wire experiences a compressive strain equal to the tensile strain, with the nominal value of both the tensile and compressive strains referred to herein as the "strain amplitude." The jaws are then rotated in concert (i.e., each jaw rotated with the same speed and in the same direction), such that the area of maximum tensile strain is rotated around the wire "peak" and transitioned to the area of maximum compressive strain with each 180-degree rotation of the jaws and wire. Rotary beam fatigue testing is further described in ASTM E2948. 14.

"Nitinol" is a trade name for a shape memory alloy comprising approximately 50 or 55 atomic % nickel (Ni), and the balance titanium (Ti), also known as NiTi, commonly used in the medical device industry for highly elastic metallic implants. For purposes of binary NiTi (e.g., NiTi compositions consisting of Ni and Ti), the Ni may comprise between 45 atomic % and 60 atomic % Ni, such as between 45 atomic % to 55 atomic % Ni, between 47 atomic % and 53 atomic % Ni, and particularly between 49 atomic % and 51 atomic % Ni; and/or between 50 atomic % and 60 atomic % Ni, such as between 53 atomic % and 57 atomic % Ni, and particularly between 54 atomic % and 56 atomic % Ni , or within any range using any two of the foregoing as endpoints. In any of the foregoing cases the Titanium may comprise the balance of the composition. "Nitinol" or "NiTi" may also refer to shape memory alloys including the nickel and titanium with additional tertiary or quaternary elements which may be desired to modify the properties of the final alloy. For purposes of the present disclosure, NiTi containing a tertiary or quaternary element may include at least 20 wt. % nickel, between 35 wt. % titanium and 55 wt. % titanium, and at least one of the following additional alloying elements:

- yttrium between 0.01 wt. % and 0.15 wt. %, such as between 0.03 wt. % and 0.12 wt. %, between 0.05 wt. % and 0.10 wt. %, or 0.07 wt. % and 0.09 wt. %, or within any range using any two of the foregoing as endpoints;
- copper between 1 wt. % and 10 wt. %, in lieu of an equal amount of nickel;
- niobium between 1 wt. % and 15 wt. %, in lieu of an equal amount of titanium;
- hafnium between 0.5 wt. % and 50 wt. %, in lieu of an equal amount of titanium;
- zirconium between 0.5 wt. % and 35 wt. %, in lieu of an equal amount of titanium;
- cobalt between 0.1 wt. % and 5 wt. %, in lieu of an equal amount of titanium, nickel, or a combination of titanium and nickel;
- chromium between 0.1 wt. % and 1 wt. %, in lieu of an equal amount of titanium; and
- iron between 0.1 wt. % and 10 wt. %, in lieu of an equal amount of titanium, nickel, or a combination of titanium and nickel; and
- tantalum between 1 and 10 wt. %.

Specifically, yttrium may be added as an additional alloying element to any of the foregoing NiTi compositions, at the amounts described above, to decrease or prevent the formation of inclusions in the NiTi during formation. Here, yttrium has been found to inhibit the formation of undesirable oxide inclusions in the alloys, which, in turn, improves the ability to process NiTi into bar and/or wire form(s) and enhances the fatigue resistance of alloy and products produced from the NiTi alloy. Therefore, yttrium may be added to the NiTi compositions at the given amounts to result in a wire with higher fatigue resistance than compositions that do not include yttrium.

Mechanical performance and nominal mechanical performance metrics, including strength, stiffness, ductility, and the like, may be evaluated for wire samples via a uniaxial tensile test. Such testing may be performed, for example, in accordance with standard ASTM E8 / E8M - 21 using an Instron Model 5565 test machine available from Instron of Norwood, Massachusetts, USA). More specifically, destructive uniaxial tension testing of the wire materials is used to quantify ultimate strength, yield strength, axial stiffness, ductility, etc. of candidate materials, using methods described in Structure-Property Relationships in Conventional and Nanocrystalline NiTi Intermetallic Alloy Wire, Journal of Materials Engineering and Performance 18, 582-587 (2009) by Jeremy E. Schaffer. These tests may be performed using servo-controlled Instron load frames in accordance with industry standards for the tension testing of metallic materials.

"Superelastic" material is material which is capable of undergoing strain exceeding 2% with negligible plastic deformation (e.g., less than, or equal to, 0.2% plastic deformation), such that the material is able to return to its original dimension with release of mechanical load after the deformation with negligible permanent damage.

"Shape Memory" material is material which is capable of undergoing strain exceeding 2% with negligible irreversible plastic deformation (e.g., less than, or equal to, 0.2% plastic deformation), such that the material is able to return to its original dimension with heating after the deformation with negligible permanent damage.

"Isothermally recoverable strain" is recoverable strain observable in a substantially constant ambient temperature, i.e., without external heating or cooling of the work piece. The work piece may experience some internal heating or cooling from microstructural changes within an isothermal strain recovery. Ambient temperature may vary by a small amount during isothermal strain recovery, such as plus-or-minus 3°C from the nominal temperature at the start of the stain recovery. Ambient temperature may be room temperature, i.e., 20-30 °C, or body temperature, i.e., 36.4-37.2°C.

"DFT®" is a registered trademark of Fort Wayne Metals Research Products Corp. of Fort Wayne, IN, and refers to a bimetal or poly-metal composite wire product including two or more concentric layers of materials, typically at least one outer layer or shell disposed over a core filament, and formed by drawing a tube or multiple tube layers over a core element.

"Impurities," "incidental impurities" and "trace impurities" are material constituents present in a material at less than 500 parts per million or 0.05 wt. %. Alloys "free" of or "excluding" a certain constituent are alloys having such a constituent in amounts equal to or less the 500 parts per million impurities threshold.

"Shape set" wires are the wires resulting from the process of "shape setting" which, as used herein, denotes a process in which a work piece (such as a wire) is constrained to a desired shape and thermally processed to retain the desired shape. For example, the work piece may be bent or otherwise formed into a desired shape, and held in that shape during subsequent thermal processing. In another example, the work piece may be constrained to its "natural" undeformed, pre-existing shape, which may include a straight shape. This "constraint" may not impart any stress to

**EP 4 569 523 B1**

the material prior to thermal processing, but rather, may simply prevent the material from deforming away from the undeformed shape during subsequent thermal processing. With the work piece so constrained, the temperature of the work piece is increased in a thermal processing step until the work piece retains the desired shape, at which point the shape setting process is completed. Additional discussion of shape set wires and the shape setting process is provided below.

1. Introduction

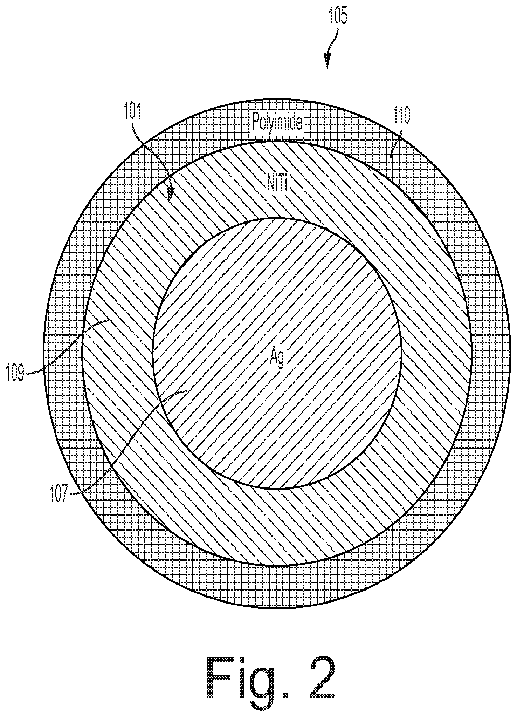

The present disclosure provides wire constructs, such as wire 105 shown in Fig. 2, which provide a high conductivity silver core 107 and a nitinol shell 109. The nitinol shell is effectively substituted for the predicate CoNiCrMo commonly used in conductor subcomponents for in cardiostimulation and neurostimulation leads. This substitution is shown to give 50 to 100% improvements in cyclic strain-loading fatigue performance for bifilar coils or monofilament wire at body temperature (310K $\pm$ 2). Additionally, wire 105 maintains the electrical isolation properties of the polyimide coating 110 even after high temperature (e.g., 450°C -550°C) shape setting occurring after the coating 110 is applied.

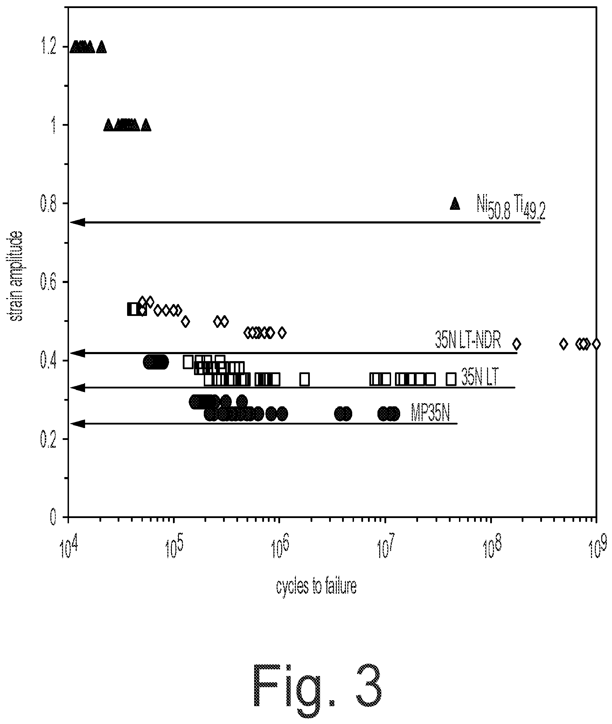

Nitinol surpasses other materials for strain-fatigue properties, as it offers low stiffness, high strength, and a unique ability to elastically recover deformations exceeding 10% strain (e.g., superelasticity). These properties have led to widespread use in guidewires and peripheral, neurovascular, and gastrointestinal stenting. The low stiffness of nitinol drives relatively low stresses for a given strain level, and the superelasticity, whether transformational or linear elastic, can make a component tolerant of extreme displacements. Nitinol wire can possess double the strain-fatigue strength of 35N LT. Fig. 3 shows fatigue strain-life data taken from Ø 0.18 mm wires that were processed using industrial medical wire standards and provides an empirical illustration of increasing strain fatigue resistance from MP35N to nitinol.

Nitinol is therefore beneficial for pacing leads due to its exceptional strain-control fatigue life. For example, NiTi may have a 10 million-cycle runout of 1% alternating strain. By contrast, a high-performance conventional lead material, such as 35N LT®, may achieve 10 million-cycle runout at less strain, such as 0.5% alternating strain or less. Substituting NiTi into current designs (with intended strain levels of substantially less than 0.5%) can make the resulting device virtually impervious to fatigue damage and, therefore, allow for effectively infinite cycles.

However, nitinol has not been commonly associated with electrical leads for medical devices. In addition to cobalt-based MP35N and 35N LT materials being generally accepted for lead applications, nitinol's elasticity and requisite heat treatments complicate the manufacture of coil constructs. One common lead design employs a bifilar coil, where each filar of the coil is electrically insulated from the other by first coating the wires with a polymer. Coated 35N LT wires may be wound around a mandrel, and plastic deformation gives stability to the coil shape. By contrast, the elasticity of nitinol complicates coil forming via plastic deformation. Nitinol can instead be thermally shape set in the desired wound shape while imparting the superelastic properties, but the temperatures required would destroy commonly used polymers (ETFE, PFA, etc.) which are necessary for electrical isolation.

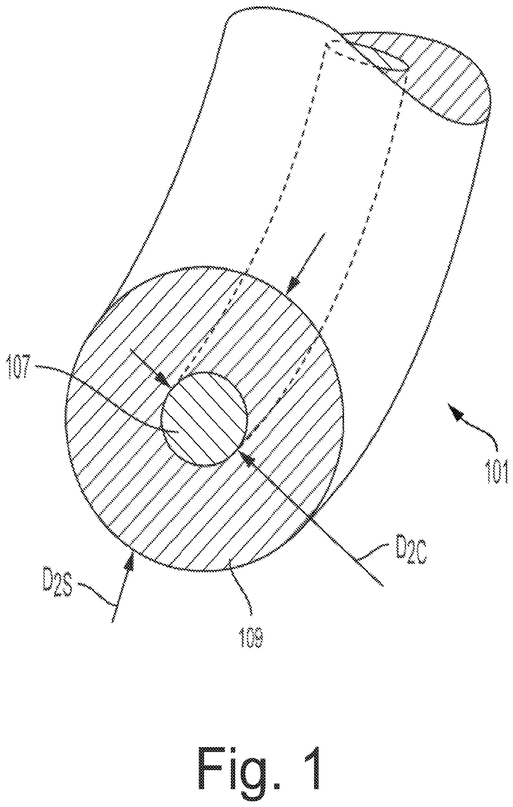

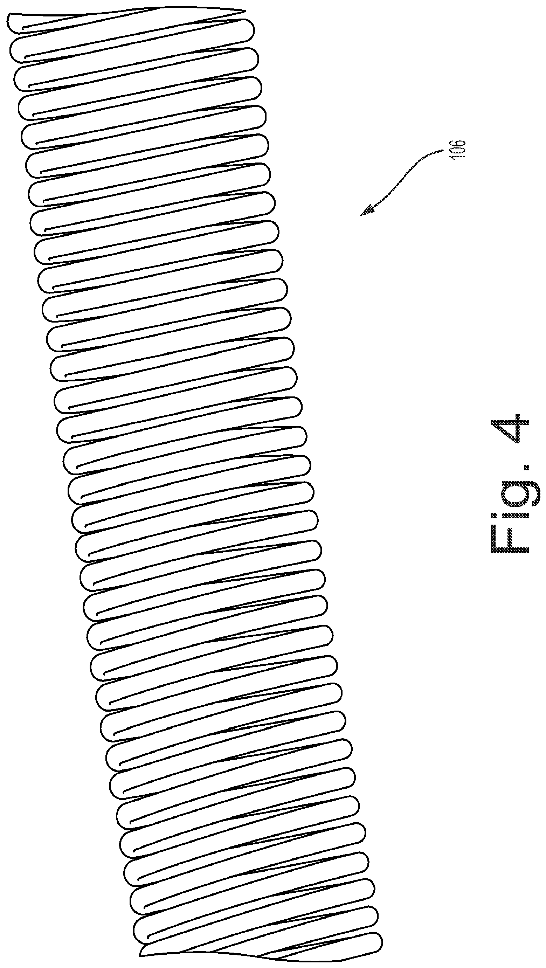

Wire 101, shown in Fig. 1 and described in further detail below, includes a nitinol component which can be coated and subsequently coiled in the manner of a traditional 35N LT design, but with the performance benefits of nitinol for coiled applications such as in coil-based leads. For purposes of the present disclosure, coils (such as coil 106, shown in Fig. 4 and described further herein) may incorporate monofilament wires such as wire 101, or may be made from multifilar constructs, such as bifilar (2-wire), trifilar (three-wire) or greater numbers of wires joined to one another. Additionally, braided or other multifilament cable constructs may also be formed into coils in accordance with the present disclosure. In one exemplary embodiment, coils in accordance with the present disclosure may be made into a Helical Hollow Strand (HHS®) type tube available from Fort Wayne Metals Research Products, LLC of Fort Wayne, Indiana.

In particular, the nitinol incorporated into wire 101 undergoes a heat treatment, generally at a temperature between 400-600°C, such as between 450°C to 550 °C, and more particularly, between 480 °C to 520 °C, or within any range using any two of the foregoing as endpoints, to shape set the wire and impart functional properties. Because typical polymers such as ETFE cannot withstand these temperatures, wire 101 utilizes an alternative polymer such as a polyimide enamel to provide excellent thermal stability and electrical resistance while also being generally regarded as safe and suitable for implantable medical devices. More particularly, the polyimide coating 110 applied to wire 101 (as seen, e.g. in Fig. 2,) and properly cured, withstands the temperatures needed to shape set nitinol in a controlled inert environment. This allows the nitinol portion of the wire 101 to be maintained in a desired twisted or wound shape, such as a coil for coil-based leads. This twisted or wound shape, in turn, enables the use of superelastic materials for new or improved designs and applications, such as pacing leads.

Wire 101 may be produced as NiTi-DFT®-Ag wire composites, i.e., having a NiTi shell 109 and an Ag core 107 (Fig. 1). As detailed below, samples of such wires were fabricated and compared to the clinically familiar 35NLT-DFT-Ag composite wires in terms of tensile, fatigue, and electrical behavior in wire, coil, and cable form.

7

2. Composite Wires with Polyimide Coating

Fig. 1 shows a composite wire 101 having a core 107 and a shell 109 surrounding the core 107. In an exemplary embodiment, core 107 and shell 109 each define longitudinal axes which are coaxial, i.e., the core 107 is centered in the shell 109. More particularly, the shell 109 defines a hollow cavity extending longitudinally along its length, and core 107 is received within and completely fills the hollow cavity, such that an inner diameter of defined by the cavity and the outer diameter of the core 107 are the same, i.e., diameter $D_{2C}$ as shown in Fig. 1. An exemplary wire 101 is DFT® available from Fort Wayne Metals of Fort Wayne, Indiana.

Composite wire 101 may be used in medical devices designed for use in the human body. If so, core 107 and shell 109 are formed exclusively of medical-grade materials, which are materials approved or otherwise suitable for use in implantable or *in vivo* medical devices. "Medical-grade" materials specifically exclude certain materials not suitable for use in, or in connection with medical procedures on, the human body. Examples of non-medical grade materials are materials not suitable for contact with tissue and/or blood, including materials which cannot pass cytotoxicity testing of at least one hour of such contact. Non-medical grade materials include heavy metals including lead and cadmium, materials such as beryllium and beryllium copper, and any other materials generally regarded as toxic to the human body or otherwise damaging to human tissue. All materials discussed herein with respect to their potential use for shell 109 or core 107 are medical-grade materials.

For example, a composite wire 101 using NiTi for the shell and Ag for the core (which may be referred to as "NiTi-DFT-Ag" wire) may be used for electrical leads. One exemplary wire design, shown in Fig. 2 and described further below is NiTi-DFT-41%Ag wire in which an Ag core 107 represents 41% of the overall cross-sectional area of wire 101, with the NiTi shell 109 representing the balance of the cross-sectional area. This wire 101 may be drawn to a desired diameter, such as 0.005", and then coated with a polymeric coating such as a polyimide 110.

Polyimides may be particularly suitable for use as a coating in composite wire 101, not only for polyimides' use as a medical grade coating, but also for its high thermal stability, making polyimides particularly suitable for coatings for shape-set alloy materials such as NiTi.

Many medical grade thermoplastic polymeric coatings are subject to thermal degradation (e.g., thermal decomposition) at relatively low temperatures. Here, these polymeric materials experience damaging chemical changes such as polymerization, side-group elimination, random chain scission, and/or oxidation of the polymer(s) at temperatures below the heat treatment temperature required for shape setting the NiTi (e.g., 400 °C to 600°C, as described previously).

Such thermal degradation may be measured by methods known in the art such as thermogravimetric analysis (TGA) whereas a sample of polymeric material is heated in a controlled atmosphere at a defined heating rate whilst the sample's mass is measured. Here, when a polymer sample degrades, the mass decreases due to the production of gaseous products such as carbon monoxide, water vapor, carbon dioxide, and the like. In this case, the thermal degradation may depend upon the amount of oxygen present in the atmosphere, where generally lower thermal degradation values are observed in controlled environments with limited oxygen (e.g., containing inert gasses) versus environments containing oxygen whereas oxidation reactions can freely occur. Thermal degradation may be reported as a degraded mass % at a given temperature for a given time interval in a certain environment (e.g., in a controlled environment containing an inert gas, such as nitrogen or argon). Thermal degradation may also be measured by differential thermal analysis (DTA) and/or differential scanning calorimetry (DSC) whereas the thermal flux (e.g., heat flow) associated with oxidation reactions are measured while heating the materials to the glass transition, melt temperature, and so on, as is known in the art.

Here, thermal decomposition (e.g., $T_{d0}$ or $T_d$ and/or T1 % values) may begin at temperatures well below 600 °C for numerous polymeric compositions, such as below 550 °C, below 500 °C, below 450 °C, below 400 °C, below 350 °C, or below 300 °C, or between any of the foregoing values used as endpoints. For example, significant thermal degradation may occur to fluorine-containing polymers, such as polytetrafluoroethylene (PTFE), below 485 °C.

Polyimide-based materials, on the other hand, may maintain thermal stability (e.g., may not thermally degrade) until temperatures well above 400 °C when tested in a controlled environment (e.g., an inert gas environment, such as nitrogen or argon gas) for a defined time interval. For instance, polyimide materials may have a thermal degradation of less than 10 mass % for temperatures as high as 400 °C, 450 °C, 500 °C, 550 °C, or 600 °C, or between any of the foregoing values as endpoints, such as between 550 °C and 590 °C, and/or between 570 °C to 580 °C, as measured when heating the material in an inert environment (e.g., in an inert gas) for a residence time of 10 minutes or less. This relatively low thermal degradation and high thermal stability makes a polyimide-based material particularly suitable for use as a coating for composite wire 101 since the polyimide avoids degradation in excess of the shape set temperatures of the NiTi material. Specifically, the polyimide material may lose less than 10% mass when heated to 500 °C for 5 minutes in an inert atmosphere (e.g., argon gas).

Polyimide 110 was coated onto composite wire 101 to an overall diameter of .006" to form coated wire 105 (Fig. 2). This coated wire 105 is then thermally treated at 600°C in an inert atmosphere (such as in Ar, He or N2 gas) to cure the polyimide coating 110 and make it able to withstand a later shape-set treatment. After curing, the coated wire 105 is then coiled around a mandrel of suitable diameter, such as 0.050" and, while still constrained in the coiled configuration, heat treated at 500°C for 5 minutes in an inert atmosphere. This second heat treatment shape-sets the NiTi, such that the wire 105 can maintain its coiled configuration without external force. This creates a coil 106, shown in Fig. 4.

The resulting coil 106 can then be used in a medical device or other application, such as an electrical lead assembly to conduct electrical power for pacing, defibrillation, sensing, neurostimulation or other implantable lead applications. In particular, coil 106 and other configurations of wire 105 are advantageously employed in applications where fatigue durability is important.

Various materials are contemplated for core 107 of composite wire 101 in the context of the present disclosure. Suitable materials may include Ag, Cu, Au, Pt, Ta, Pd, Nb, Mo, and Al, as well as alloys thereof, with material choice largely dependent on the intended final use or application of the composite wire 101. Core ratios may be between 10-95% for various applications. For many applications, core ratios between 20-65% may be employed.

For purposes of illustrating the properties of coated wire 105 relative to predicate designs, a reference composite wire was produced with a 35N LT shell and pure silver (99.99% purity) core by precision machining the shell material to a tubular form, conditioning the material to provide smooth surfaces free from contamination and debris, then filling the tubular materials with high purity silver and co-processing to form silver-core monofilament DFT (drawn filled tube) wires. In the resulting bimetal reference wire, the silver core represented 28% of the cross-sectional area of the wire, and this composite is designated herein as 35NLT-DFT-28Ag. Cold work reduction and annealing processes were conducted as described in detail below to create a finished reference wire.

For comparison to the reference wire, a composite wire 101 with shell 109 made of nitinol and a core 107 made of pure silver was produced in a similar manner as described above. For this example of wire 101, the core percentage was slightly larger at 30% but still within typical tolerances. This composite is designated as NiTi-DFT-30Ag. Nitinol used here was $Ni_{50.8}Ti_{49.2}$ (at.%) extra low interstitial grade material with an ingot austenitic peak temperature (Ap) of 248 K (SE508 ELI grade, Confluent Medical, Scottsdale, AZ) as defined by ASTM F2004-17.

Both the reference wire and the composite wire 101 described in the foregoing paragraph were processed down to testable configurations given in Table 1. In Table 1, bifilar coils are denoted as 2 wires x 0.100 mm each coated with polymer and coiled to 0.76 mm OD. Strands are given as 1 x 19, meaning 19 concentrically wound filaments forming a single multifilament cable. In Table 1, these 1 x 19 cables are formed from 0.025 mm filaments giving with a nominal cable diameter of 0.125 mm.

Table 1. Wire-based DFT wire constructs assessed for comparison.

| Wire | Material | Type | Size/Configuration |
|---|---|---|---|
| 1<br>2 | 35N LT-DFT-28%Ag<br>NiTi-DFT-30%Ag | Monofilament wire | 0.100 mm |
| 3<br>4 | 35N LT-DFT-28%Ag<br>NiTi-DFT-30%Ag | 1x19 Stranded wire | 1x19x0.025; 0.125 mm |
| 5<br>6 | 35N LT-DFT-28%Ag<br>NiTi-DFT-30%Ag | ETFE-coated Bifilar coiled wire<br>Polyimide-coated Bifilar coiled wire | 2 x 0100/0147; 0.76 mm<br>2 x 0.100/0.114; 0.76 mm |

For the reference wire in Table 1 (shown as Wire 1), a coating of ethylene tetrafluoroethylene (ETFE) was applied by crosshead extrusion. Wire 1 prior to the coating was a single 0.100 mm diameter 35NLT-DFT-28Ag wire as noted above. After coating, the reference wire had a total diameter of 0.147 mm giving a polymer wall thickness of 24 microns.

For wire 101 in the present comparison, shown as Wire 2 in Table 1, Pyre-M.L. (RC5019, Industrial Summit Technology, Otsu, Japan) polyimide (PI) enamel was applied, and heat cured layer-by-layer to achieve a coated diameter of 0.114 mm over the initial diameter of 0.100 mm, a polymer wall thickness of 7 microns.

Bifilar (2-wire) coils of both 0.1 mm coated 35NLT-DFT-28Ag and NiTi-DFT-30Ag were formed using tubular strand equipment to form to a final OD of 0.76 mm. For purposes of the present disclosure, bifilar wires are simply a pair of wires having their insulating coatings bonded while their electrically conductive bimetal wires remain electrically isolated from one another. The reference bifilar wire made from 35NLT-DFT-28Ag wires was cold-formed to the mandrel and removed as a stable coil without using heat to stabilize the shape. This coil is represented as Wire 5 in Table 1.

NiTi-DFT-30Ag bifilar coils were made from a pair of wires 101 bonded by contact between their respective coatings 110. After coiling, these bifilar coils required heating to about 773 K for seconds to stress relieve the nitinol, reverting the SMA backbone from strained martensite (as-coiled) to austenite parent phase, thereby shape setting the coiled form prior to mandrel removal. This coil is represented as Wire 6 in Table 1.

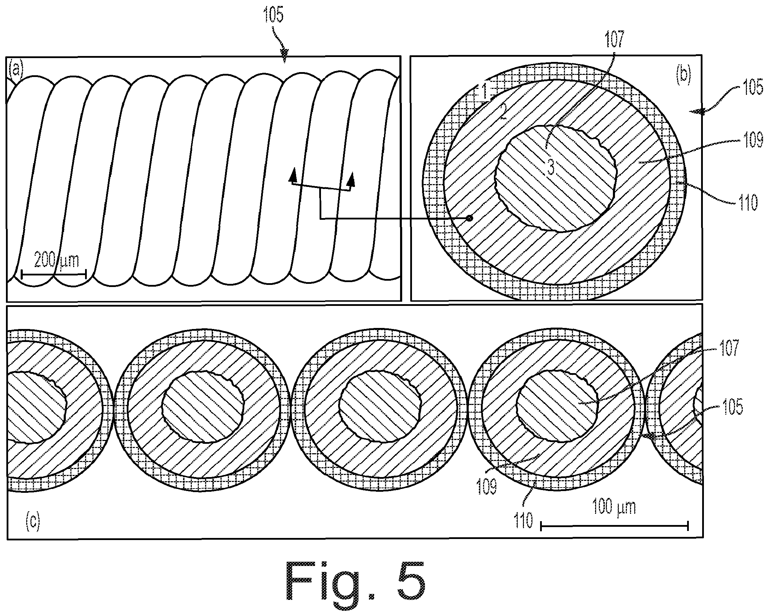

Fig. 5 shows the surface and cross-sections of polyimide-coated, strand-coiled and shape set DFT Wire 6 of Table 1 in (a) secondary electron imaging of the coated coil surface; (b) a singular wire section showing the distinct wire ingredients; and (c) multiple sections of coil elements with adjacent-wire polymer isolation after complete thermal processing. Fig. 5(a) is drawn to scale as shown in the lower-left of that portion of the figure; Fig. 5(c) is drawn to scale as shown in the lower-right of that portion of the figure. As shown in Figs. 5(b) and 5(c), the polyimide coating 110, the superelastic nitinol mechanical backbone forming shell 109, and the high purity, high conductivity 99.99% silver core element 107 were all maintained to a high degree of concentricity and dimensional stability even after coiling and heat treatment.

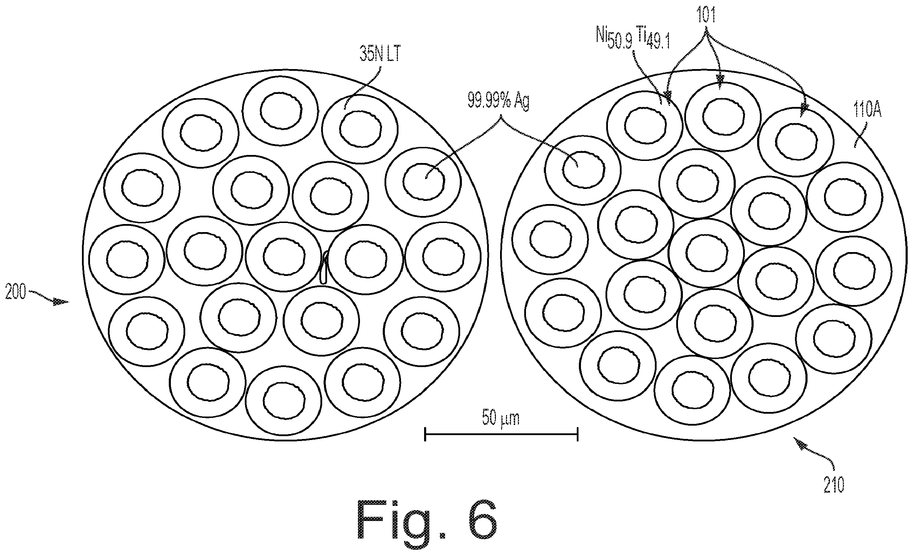

Fig. 6 shows metallographically prepared cross-sections of Wires 3 and 4 in Table 1, drawn to scale according to the scale shown in the lower-center of the figure. Both reference wires and wires 101 were formed into a 1 x 19 by 25 $\mu$m DFT-wire cable configuration with total outside diameters of about 125 $\mu$m, with the reference cable shown as cable 200 at the left side of Fig. 6 and the cable incorporating wires 101 shown as cable 210 at the right side of Fig. 6. For cable 210, wires 101 are all surrounded by a common insulating layer 110A rather than being individually insulated as shown and described herein with respect to monofilament and bifilar wires. As illustrated, insulating layer 110A provides a continuous layer of insulation around each of the wires 101 to provide for electrical isolation between and among all the individual wires 101. Insulating layer 110A may be a similar composition to Polyimide 110, as described in reference to FIG. 2. For purposes of comparison, cable 200 is similarly constructed.

The chosen concentric strand configuration for cables 200 and 210 is representative of conductors used in some neurological and cardiac stimulation leads and were produced as concentric 1 x 19 configurations in both 35NLT-DFT-28Ag (Wire 3) and NiTi-DFT-30Ag (Wire 4). In both configurations, monofilament 25 $\mu$m wires were concentrically stranded first as a layer of six wires over one to create a 7-wire wind, and then 12 wires over the 7-wire wind, to give 18 helixed conductor wires around one straight core element. Both strand types were thermally stress relieved for less than 10 minutes at 773 to 973 K using a reel-to-reel process to stabilize the multifilament wires against fray at cut ends.

The wires shown in Table 1 are used for illustration, it being understood that other forms, configurations and sizes may be made in accordance with the present disclosure. For example, wire 101 may be made in a variety of sizes, ranging from ultrafine wire having an overall diameter $D_{2S}$ of as little as 20 $\mu$m, 30 $\mu$m, 40 $\mu$m or 50 $\mu$m, or between any of the foregoing values used as endpoints, to fine wire having an overall diameter $D_{2S}$ between 100 $\mu$m, 150 $\mu$m or 250 $\mu$m, or between any of the foregoing values used as endpoints, for example. For applications (e.g., electrical leads) using multifilament constructs, such as strands and cables, overall diameter $D_{2S}$ may be between 20-50 $\mu$m. For applications (e.g., electrical leads) using coils, overall diameter $D_{2S}$ may be commonly between 80-120 $\mu$m.

Similarly, the monofilament, bifilar, and cable configurations of the wires shown in Table 1 are used for illustration, with other configurations contemplated within the scope of the present disclosure. In terms of cables, 1 x 19 is one configuration among many possible configurations. Other cable configurations are discussed in International Application No. WO 2018/183862 A1, entitled SMALL DIAMETER CABLE and filed March 31, 2017,

3. Coated Composite Wire Performance

Tensile testing was conducted on the various wire samples in Table 1 to establish comparative metrics for reference wires and wires made in accordance with the present disclosure. Monotonic uniaxial tensile properties were measured for each material at 298 K at a strain rate of 10-3 s-1 with pneumatic grips and flat grip faces lined with fine 600-grit emery to prevent sample slippage.

Wire resistance testing was performed using a temperature-compensated 4176 Precision Micro-Ohmmeter (Valhalla Scientific) to assess direct current (DC) resistance over a 304.8 mm straight wire section. Diameters for use in resistance and tensile stress calculations were measured using a high precision bench micrometer equipped with a Dorsey gage (0.00005 inch graduations, Dorsey Metrology International and 673MXZ bench micrometer, L.S. Starrett Co).

Rotary beam fatigue testing in accordance with ASTM E2948 was conducted on 0.1 mm Wires 1 and 2 to assess basic material level fatigue life. Fatigue life was assessed at various strain loads using equipment manufactured by Positool, Inc. at 60 Hz in body temperature 310K ($\pm$ 2) 0.9% saline with a strain load ratio of R = -1. The monofilament specimens were tested at alternating strain levels ranging from 0.5 to 2.5% to fracture or to a runout criterion of $10^8$ cycles.

Coil flexural fatigue testing of Wires 5 and 6 in Table 1 was conducted by rotary beam methods using the same Positool rotating chuck apparatus. In this test, the minimum free-loop chuck and rest geometry governed the maximum achievable outer fiber wire alternating strain to about 0.40%. Under this strain-load level at 60 Hz in body temperature saline, neither coil configuration fractured over 10 samples within the $10^8$ cycle runout criteria and tensile-tensile fatigue testing was commenced to induce a more severe material strain condition.

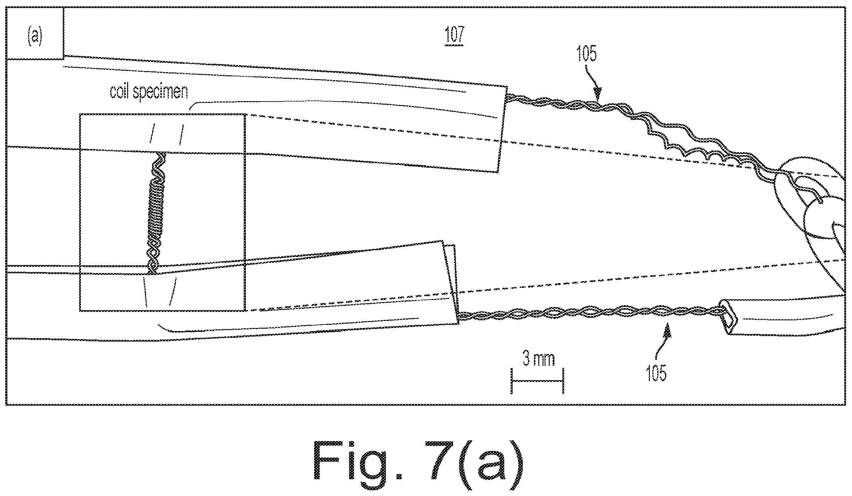

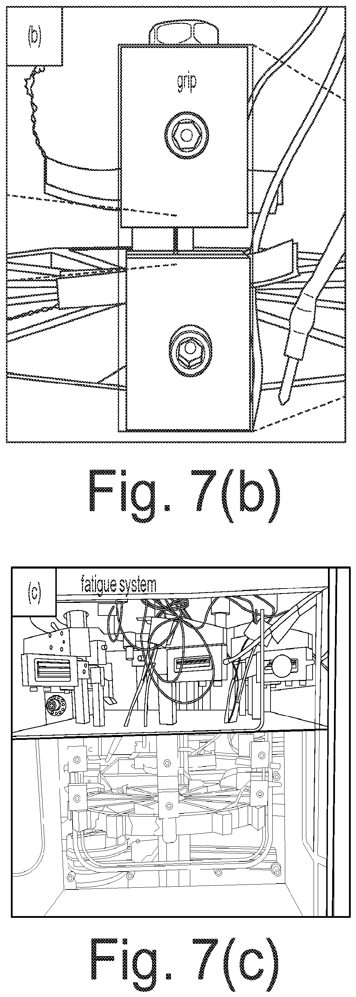

Coil tensile-tensile fatigue testing was performed using 3.5 mm discrete specimens of Wires 5 and 6 in Table 1. These wires were loaded in body temperature distilled water on a Bose Enduratec ELF 3230 system (TA Instruments, Eden Prairie, MN) with custom wire grip, continuity, and load sensing (Element Minneapolis, Minnetonka, MN). Compliant sections of coated bi-filar material were formed at a gage length of 3.5 mm with continuous stretched support sections for electrical tape padding (Fig. 7a) before loading into flat face steel grips (Fig. 7b) and further loading into the distilled-water-immersed fatigue system (Fig. 7c) with 6 parallel samples per test setup. All fixtured parts and grips were aligned and secured before final loading and cycling at a rate of 35 Hz at a bath temperature of 310K ($\pm$ 2). Displacement conditions for cycling were 7 mm mean displacement and levels of $\pm$ 0.35, $\pm$ 0.53 and $\pm$ 0.75 mm giving structural strains of 200% mean and alternating structural strains of 10%, 15% and 21.4% respectively. Specimens were cycled until fracture or a $10^8$-cycle runout criterion.

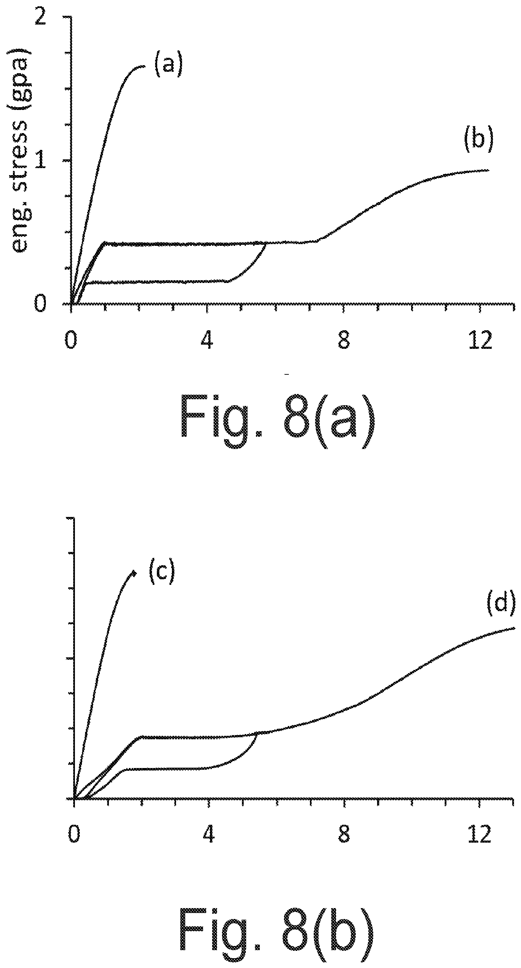

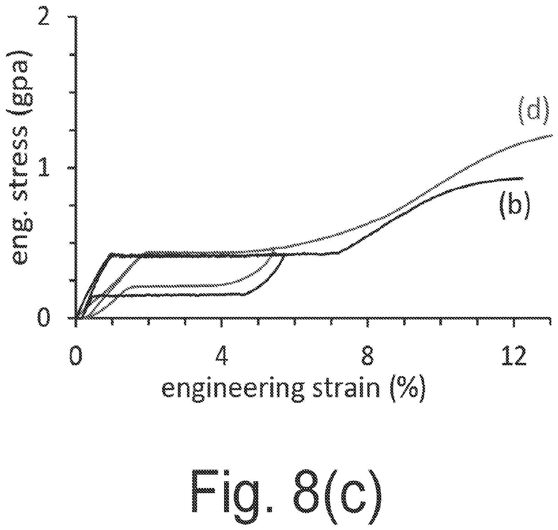

Fig. 8(a) shows the tensile behavior of Ø 0.1 mm monofilament wires, with the reference wire (i.e., Wire 1 of Table 1) shown at curve (a) and wire 105 in a 0.1 mm NiTi-DFT-30Ag wire configuration (i.e., Wire 2 of Table 1) shown at curve (b). Fig. 8(b) shows the tensile behavior of Ø 0.125 mm 19-wire strands. The reference cable made with wire of 35NLT-DFT-28Ag strands for a total diameter of 0.125 mm (i.e., Wire 3 of Table 1) is shown at curve (c) and a cable made with wires 105 forming NiTi-DFT-30Ag strands for a total diameter of 0.125 mm (i.e., Wire 4 of Table 1) shown at curve (d).

An ultimate strength and elongation to fracture of about 1600 MPa and 2.8% strain respectively was observed for Wire 1 of Table 1, on par with similar wires used in the medical device industry. In the multifilament, reference cable shown in Fig. 6 and at curve (c) in Fig. 5(b), Wire 3 of table 1 was measured at about 90% of the strength and elongation of monofilament wire, shown at curve (a) of Fig. 5(a), also par for similar commercial product test outcomes.

The nitinol-based monofilament wire 105, generated curve (b) in Figs. 5(a) and 5(c), the and the multifilament, cable 210 generated curve (d) in Figs. 5(b) and 5(c). As illustrated, wire 105 and cable 210 exhibited lower strength levels (about 30%) and much higher elasticity compared to their reference 35N LT counterparts due to pseudoelastic isothermal strain recoverability. Elastic modulus under initial loading to about 200 MPa, prior to transformation onset, was reduced for the nitinol-based cable 210 as compared to the wire 105, as can be seen by a comparison of curve (d) vs. curve (b). In monotonic tensile loading to fracture, the work energy to fracture averaged 23.7 and 65.9 mJ/mm$^3$ for 35NLT-DFT-28Ag and NiTi-DFT-30Ag respectively.

Thus, while the ultimate strength of the nitinol-silver composite wire 105 at about 1000 MPa was lower than CoNiCrMo-silver composite reference wire at about 1600 MPa, the work energy to tensile fracture was 65.9 mJ/mm3 for nitinol-silver and 23.7 mJ/mm3 for CoNiCrMo-silver, requiring significantly more energy to overload the nitinol-based conductor. Generally speaking, wire 105 made in accordance with the present disclosure can be expected to exhibit at least twice the work energy to tensile fracture compared to the same wire configuration substituting CoNiCrMo materials, such as 35NLT, for the NiTi of wire 105. For purposes of comparison, the "same wire configuration" implies that both wires would be identical except for the material of the shell, with the same overall diameter, core material(s) and core ratio.

Cable 210 and the reference cable were also tested for electrical performance. The results of DC resistance testing for the 1 x 19 strand configurations are given in Table 2, which shows similar mean values for either material. At a mean of 5.99 ohm/m, the NiTi-DFT cable 210 made in accordance with the present disclosure was about 5% more conductive that the 35NLT-DFT reference cable, consistent with the slightly larger core in the NiTi-DFT wire and a minor contribution from higher conductivity shell material. Moreover, Table 2 also illustrates no degradation of the electrical insulation provided by coating 110A (Fig. 6) after final shape setting.

Table 2. DC Resistance test summary of stranded DFT wires.

| | Strand Ø [mm] | Resistance [ohm/m] | | Strand Ø [mm] | Resistance [ohm/m] |
|---|---|---|---|---|---|
| 35N LT-DFT Stranded Wire | 0.1288 | 6.32 | NiTi-DFT Stranded Wire | 0.1257 | 5.99 |
| | 0.1290 | 6.32 | | 0.1255 | 5.98 |
| | 0.1288 | 6.32 | | 0.1255 | 5.98 |
| | 0.1290 | 6.31 | | 0.1257 | 5.98 |
| | 0.1285 | 6.33 | | 0.1255 | 6.00 |
| | 0.1290 | 6.33 | | 0.1255 | 6.00 |
| mean | 0.1289 | 6.32 | mean | 0.1256 | 5.99 |
| SD | 0.00021 | 0.0067 | SD | 0.00013 | 0.0089 |

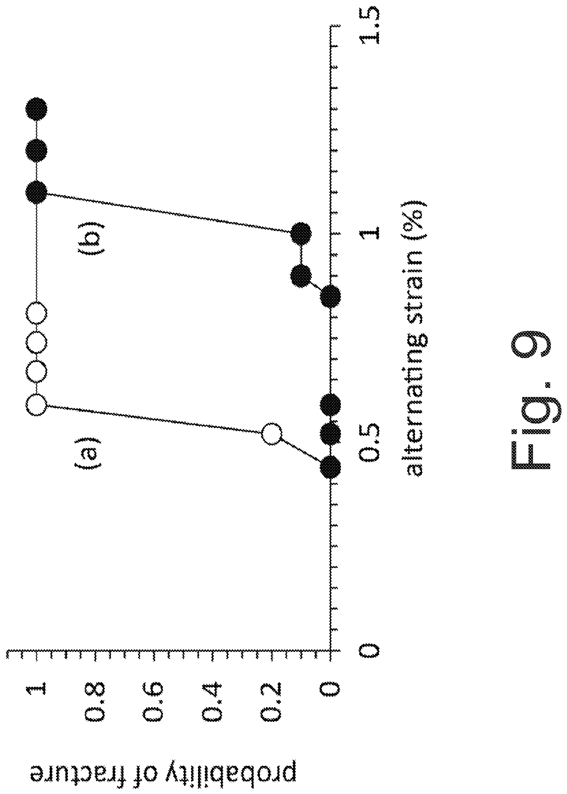

Fig. 9 shows the empirical probability of fracture as a proportion of 10 samples each of Wires 1 and 2 that fractured prior to the $10^8$-cycle runout criterion in strain-based R = -1 rotating beam fatigue in 310K 0.9% saline solution. Probabilities were empirically derived from sample size 10 per alternating strain level at R = -1 strain load ratio for Ø 0.1 mm 35NLT-DFT-28Ag reference wire, shown at curve (a), and for Ø 0.1 mm NiTi-DFT-30Ag wire 105, shown at curve (b). As illustrated, from zero to fifty percent probability of fracture the NiTi-DFT-30Ag wire 105 showed a 90 to 100% increase in strain-load capability as compared to the 35NLT-DFT-28Ag reference wire, which reached 100% probability of fracture at 0.5% while the wire 105 had an equivalent fracture probability at 1.1% alternating strain.

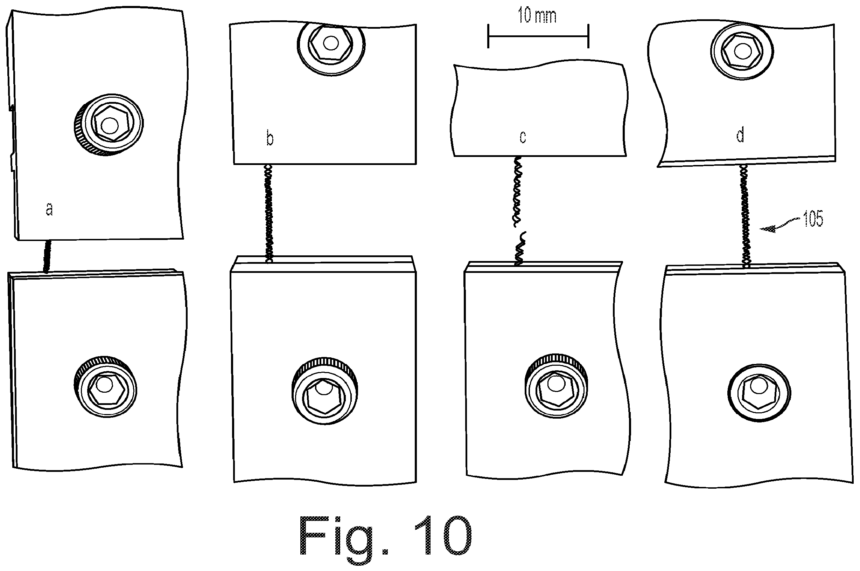

Turning to Fig. 10, bifilar coil samples fixtured in the testing system of Figs. 7(a)-7(c) are shown. A reference 35NLT-DFT-28Ag bifilar sample is shown at Figs. 10(a)-10(c), and a NiTi-DFT-30Ag bifilar sample made in accordance with the present disclosure is shown at Fig. 10(d) before and after cycling.

The initial 3.5 mm gaged grip separation is shown in panel (a), and 200% structural strain extension to a mean 10.5 mm gage is shown in panel (b), both for the 35N LT-based cohort. Panel (c) shows typical fracture of 35NLT-DFT-28Ag whereas panel (d) shows typical survival of NiTi-DFT-30Ag after more than 20 million cycles.

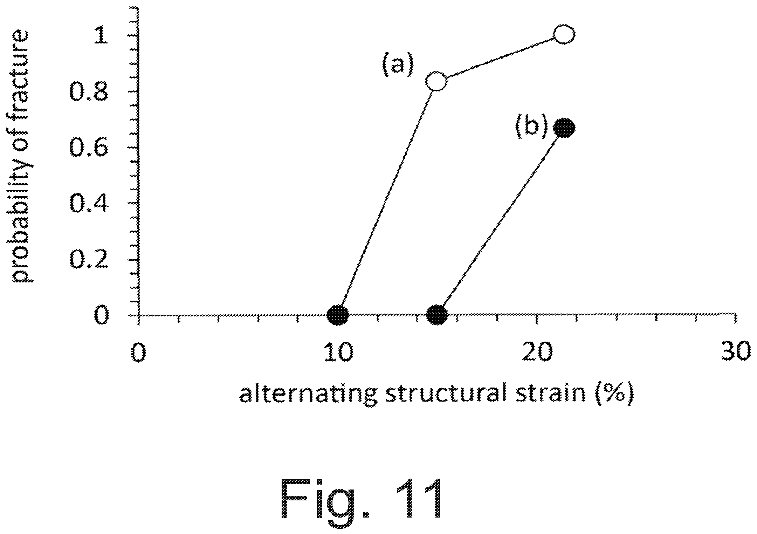

Fig. 11 provides a plot of empirical probability of fracture from N = 6 sampling of both the reference material type and the bifilar material including wires 105. Both samples were tested under coil-axial tension-tension fatigue at 200% mean structural strain and varied levels of structural strain conducted in 310 $\pm$ 2K distilled water.

Bifilar 35NLT-DFT-28Ag reference wire survived to a total of $10^7$ cycles at 10% structural strain cycling with five of six samples breaking in similar fashion to Figure 10(c) during cycling at 15% structural strain. In contrast, NiTi-DFT-30Ag bifilar wire including wires 105 showed zero fracture during 15% structural strain cycling whereas two of six samples survived to a total of 3 x $10^7$ cycles during cycling at 21.4% structural strain.

Further compositions of NiTI-DFT-30Ag including amounts of the additional alloying element yttrium as discussed previously (e.g., from 0.01 to 0.15 wt. % of the composition of NiTi) are tested in the same manner as those described relating to NiTi-DFT-30Ag above. Here, samples prepared from NiTi-DFT-30Ag containing yttrium demonstrate similar, or better runout performance (e.g., higher runout cycles at the tested alternating strain values) than those containing no yttrium.

## 4. Drawing and Cold Work

In an exemplary method of production, a hollow tube is procured in the desired materials for shell 109. A core material is inserted into the hollow tube such that the interior cavity of the tube is completely filled by the core. This intermediate, or pre-form, material is then formed into a coarse wire structure by, for example, a schedule of drawing and annealing the intermediate material to create a structure ready for final processing into wire 101. Thereafter, the coarse wire structure may be subjected to one or more additional draws, as well as a final cold work conditioning step to form the finished and final construct, namely wire 101. One or more thermal processing steps including shape setting, annealing and/or aging, as described herein, may then be performed in order to impart desired mechanical properties to the finished wire product, including superelasticity as discussed above. Further details of exemplary wire production and processing methods are further described below.

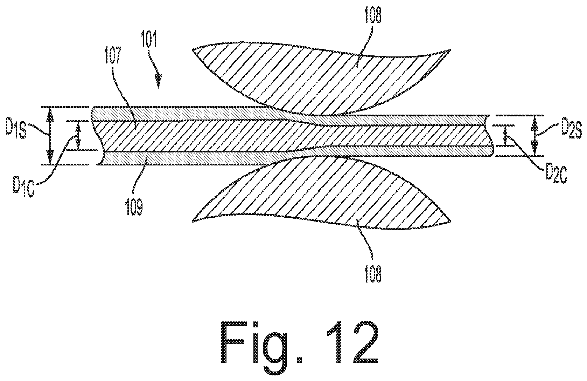

In one exemplary embodiment shown in Fig. 12, wire 101 made of medical-grade materials (described above) may be produced from a pre-form material into a wire of a desired diameter prior to final processing. That is, the pre-form material is drawn through one or more dies 108 (Fig. 12) to reduce the outer diameter of the intermediate material slightly while also elongating the material, after which the material is annealed to relieve the internal stresses (i.e., retained cold work as discussed below) imparted to the material by the drawing process. This annealed material is then drawn through one or more new dies 108 with a smaller finish diameter to further reduce the diameter of the material, and to further elongate the material. Further annealing and drawing of the material is iteratively repeated until the material is formed into a drawn wire construct ready for final processing into wire 101.

In an exemplary method of production, a tapered end is provided in the pre-form tubular structure made of the material selected for shell 109. This free end is placed protruding through the drawing die 108 and is then gripped and pulled through the die 108 to reduce the diameter of the construct and compresses the inner surface of shell 109 into the core 107. More particularly, the initial drawing process reduces the inner diameter of shell 109, such that shell 109 closes upon the outer diameter of core 107 and the inner diameter of shell 109 equals the outer diameter of core 107. After this initial drawing, the powder of the inner core 107 completely fills the central cavity of the outer shell 109 when viewed in section, as shown in Fig. 1. This drawing process is then iteratively repeated to further reduce the diameter of the material, which also further elongates the material. Iterative annealing and drawing of the material is performed until the material is formed into a drawn wire construct ready for final processing into a drawn composite wire 101. Further detail regarding the construction and geometry of a composite wire in accordance with the present disclosure can be found in U.S. Patent Nos. 7,420,124, 7,501,579 and 7,745,732, filed September 13, 2004, August 15, 2005 and January 29, 2009 respectively and all entitled DRAWN STRAND FILLED TUBING WIRE,

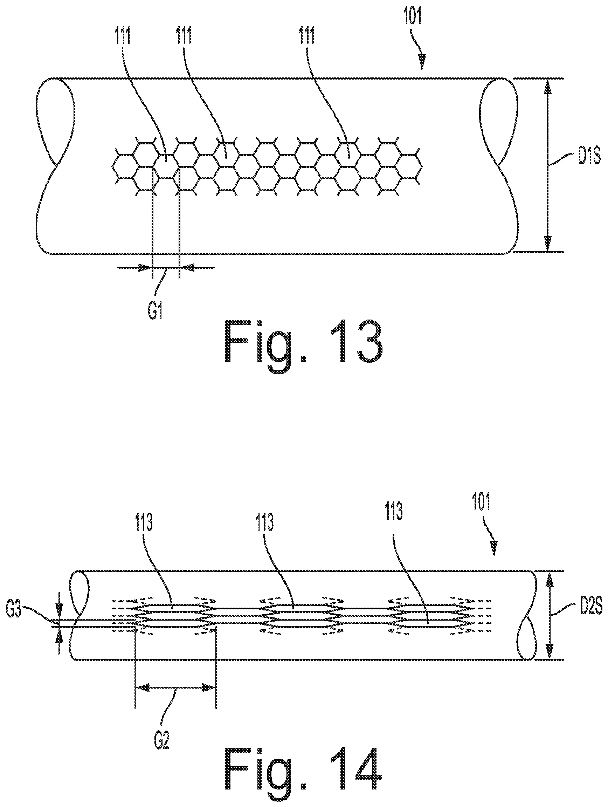

Drawn wire constructs are structurally distinguished from constructs formed by other methods (e.g., casting, machining, coating, etc.) by their characteristic smoothness and high reflectivity. In the case of the composite wire 101 described herein, the circularity of the cross-section and the concentricity of the shell and core are substantially finer in a drawn construct as compared to, e.g., a coated construct. In addition, the microstructure of a drawn construct may be structurally distinct from other constructs, for example by exhibiting an elongated grain structure (shown in Fig. 14 and further discussed below) or a fine-grain structure after thermal processing.

The step of drawing subjects wire 101 to cold work. For purposes of the present disclosure, cold-working methods effect material deformation at or near room temperature, e.g. 20-30 °C. In the case of composite wire 101, drawing imparts cold work to the material of both shell 109 and core 107, with concomitant reduction in the cross-sectional area of both materials. The total cold work imparted to wire 101 during a drawing step can be characterized by the following formula (I):

$$cw = 1 - \left(\frac{D_2}{D_1}\right)^2 \times 100\% \quad (I)$$

wherein "cw" is cold work defined by reduction of the original material area, "$D_{2C}$" and "$D_{2S}$" are the outer cross-sectional diameters of the core 107 and the shell 109 respectively after the draw or draws, and "$D_{1C}$" and "$D_{1S}$" are the outer cross-sectional diameters of the core 107 and the shell 109 respectively prior to the same draw or draws. Generally speaking, "$D_2$" as used in formula I means either $D_{2C}$ or $D_{2S}$, depending on whether cold work cw is being computed for the core or shell respectively, and "$D_1$" as used in formula I means the corresponding $D_{1C}$ or $D_{1S}$.

Referring to Fig. 12, the cold work step may be performed by the illustrated drawing process. As shown, wire 101 is drawn through a lubricated die 108 having an output diameter $D_{2S}$, which is less than diameter $D_{1S}$ of wire 101 prior to the drawing step. The outer diameter of wire 101 is accordingly reduced from pre-drawing diameter $D_{1S}$ to drawn diameter $D_{2S}$, thereby imparting cold work cw.

Alternatively, net cold work may be accumulated in wire 101 by other processes such as cold-swaging, rolling the wire (e.g., into a flat ribbon or into other shapes), extrusion, bending, flow forming, pilgering or cold-forging. Cold work may also be imparted by any combination of techniques including the techniques described here, for example, cold-swaging followed by drawing through a lubricated die finished by cold rolling into a ribbon or sheet form or other shaped wire forms. In one exemplary embodiment, the cold work step by which the diameter of wire 101 is reduced from $D_{1S}$ to $D_{2S}$ is performed in a single draw and, in another embodiment, the cold work step by which the diameter of wire 101 is reduced from Dis to $D_{2S}$ is performed in multiple draws which are performed sequentially without any annealing step therebetween.

For processes where the drawing process is repeated without an intervening anneal on composite wire 101, each subsequent drawing step further reduces the cross section of wire 101 proportionately, such that the ratio of the sectional area of shell 109 and core 107 to the overall sectional area of wire 101 is nominally preserved as the overall sectional area of wire 101 is reduced. Referring to Fig. 12, the ratio of pre-drawing core outer diameter $D_{1C}$ to pre-drawings shell outer diameter $D_{1S}$ is the same as the corresponding ratio post-drawing. Stated another way, $D_{1C}/D_{1S} = D_{2C}/D_{2S}$.

Further details regarding wire drawing are discussed in U.S. Patent Application Serial No. 12/395,090, filed February 27, 2009, entitled "Alternating Core Composite Wire", assigned to the assignee of the present invention, Drawing apparatuses and techniques are further described in International Patent Application No. WO 2019217350, filed May 7, 2019 and entitled APPARATUS AND METHOD FOR METAL-MEDIATED CATALYSIS,

## 5. Annealing

Thermal stress relieving, otherwise known in the art as annealing, is achieved by heating the material to a nominal temperature not exceeding the melting point of the material or materials used in the construct. Annealing is used to improve the ductility of the construct between drawing steps, thereby allowing further plastic deformation by subsequent drawing steps. When calculating cold work cw using formula (I) above, it is assumed that no anneal has been performed subsequent to the process of imparting cold work to the material.

Heating wire 101 to a temperature sufficient to cause recrystallization of grains eliminates accumulated cold work in solid metallic materials such as shell 109. The cold work imparted by each iterative cold work process is relieved by fully annealing the material between draws, thereby enabling the next iterative cold working process for materials which might otherwise become brittle by repeated draws or other cold work processing. In full annealing, the cold-worked material is heated to a temperature sufficient to substantially fully relieve the internal stresses stored in the material, thereby relieving the stored cold work and "resetting" cold work to zero.

On the other hand, wire 101 subject to drawing or other mechanical processing without a subsequent annealing process retains an amount of cold work. The amount of retained cold work depends upon the overall reduction in diameter from $D_{1S}$ to $D_{2S}$, and may be quantified on the basis of individual grain deformation within the material as a result of the cold work imparted. Referring to Fig. 13, wire 101 is shown in a post-annealing state, with grains 111 shown substantially equiaxed, i.e., grains 111 define generally spheroid shapes in which a measurement of the overall length G1 of grain 111 is the same regardless of the direction of measurement. After drawing wire 101 (as described above), equiaxed grains 111 are converted into elongated grains 113 (Fig. 14), such that grains 113 become longitudinal structures defining an elongated grain length G2 (i.e., the longest dimension across grain 113) and a relatively shorter grain width G3 (i.e., the shortest dimension across grain 113). The elongation of grains 113 results from the cold working process, with the longitudinal axis of grains 113 generally aligned with the direction of drawing, as illustrated in Fig. 14.

The retained cold work of wire 101 after drawing can be expressed as the ratio of the elongated grain length G2 to the width G3, such that a larger ratio implies a grain which has been "stretched" farther and therefore implies a greater amount of retained cold work. By contrast, annealing wire 101 after an intermediate drawing process recrystallizes the material, converting elongated grains 113 back to equiaxed grains 111 and "resetting" the retained cold work ratio to 1:1 (i.e., no retained cold work).

For the above-described solid metals and solid metal alloys used for shell 109, full annealing or stress-relief annealing sufficient to tune strength and straightness properties may be accomplished at a temperature between and a time dependent on material and the geometry of the wire, such as the outer diameter $D_{2S}$ of wire 101. Higher temperatures are associated with full annealing and lower temperatures associated with stress-relief annealing that does not fully recrystallize elongated grains 113 back to equiaxed grains 111. Annealing time, also called the "dwell time" during which the wire is exposed to the annealing temperature, is dependent on the size of the wire 101 and the desired effect of the annealing process, as well-understood by a person of skill in the art of material processing.

For purposes of the present discussion, annealing time may be assumed to be positively linearly correlated with the cross-sectional area of the wire being annealed. Thus, for a given annealing temperature, a similar annealing result is assumed for a first wire having a first cross-sectional area and annealed for a first amount of time, as for a second wire having twice the cross-sectional area of the first wire and annealed for a second amount of time that is twice the first time. However, for smaller fine wires and ultrafine wires, such as those having 200 μm or less, it may be assumed that the wire material becomes quickly heated through to the desired temperature, and the time for this heating is not significantly diameter-dependent. Thus, for wires 101 having diameters $D_{2S}$ less than 200 μm, the annealing time is not correlated to diameters $D_{2S}$ and is instead solely determined on the desired effect, i.e., full annealing or various gradations of stress-relief annealing as described above.

Moreover, annealing parameters can be expected to vary for varying wire diameters and wire configurations, with smaller diameters shortening the time of anneal for a given temperature and a given wire material. Whether a full anneal has been accomplished for any given wire sample can be verified in a number of ways as well known in the art, such as microstructural examinations using scanning electron microscopy (SEM), mechanical testing for ductility, strength, elasticity, etc., and other methods. Notably, the annealing parameters will affect the conditions of the core 107, but the core 107 will not experience cold work and grain deformation in the same way because the grains of the powder are free to rearrange around one another during cold work processing.

Further discussion of cold working and annealing methods can be found in U.S. Patent No. 8,840,735, filed September 18, 2009 and entitled FATIGUE DAMAGE RESISTANT WIRE AND METHOD OF PRODUCTION THEREOF.

6. Shape Setting

Shape setting may be performed on annealed material with no stored cold work with the scope of the present disclosure. However, shape setting materials with stored cold work produces larger recoverable strain capabilities for a given material geometry and constituency of the present alloys.

In the shape setting process, the work piece is subjected to an ambient temperature (e.g., in an oven or other heater) between 300°C and 600°C for a time period between 1 second and 1 hour or more. In particular, the temperature for this primary shape set be as little as 250°C, 350°C, or 400°C, and as much as 500°C, 550°C, or 600°C, or may be any temperature in any range defined by any two of the foregoing values. The temperature at this stage may be held for as little as 1 second, 1 minute, or 5 minutes, and as much as 10 minutes, 60 minutes, or 24 hours or may be any period of time in any range defined by any two of the foregoing values.

As with annealing, time and temperature are inversely correlated in a shape setting process in accordance with the present disclosure. That is to say, shape setting at the upper range of acceptable temperatures will require a generally shorter time for a given work piece geometry (e.g., size and configuration), while the lower range of acceptable temperatures will require a relatively longer time.

7. Applications

Nitinol composite wires such as wire 105 can be employed in biostimulation lead constructs to enable new and enhanced device designs. For example, pacing and defibrillation designs may include hybrid systems incorporating implantable wireless pulse generators exterior to the heart with short transmyocardial leads. Such hybrid systems could take advantage of seed-sized leadless appliances without consuming precious internal atrial or ventricular chamber space, especially in younger device recipients that may require repeat implantation. Such heart wall-crossing leads would necessarily require high myocardial compliance matching while maintaining electrical integrity through hundreds of millions of cycles of implant life. Better compliance matching with high backbone strength and enhanced cyclic displacive durability may also help avoid lead fracture issues near suture anchor points and difficult anatomical geometries found in conventional lead uses. Wire 105 is well-suited to such applications.

A deployable lead system based on wire 105 may also include programmable shape set features to provide clinical fixation benefit. For example, passive anchoring of some leads is accomplished today by creating wave or helical features that create gentle vessel apposition, as for example in the coronary sinus for cardiac resynchronization therapy. A nitinol-based lead system with thermally stable polymers, including wire 105, can be programmed to a deployable shape beneficial to gentle vessel or tissue anchor purposes providing gentle delivery, stable anchoring and simplified retrieval compared to active fixation.

The use of nitinol-based conductors such as wire 105 into neurostimulation and cardiac stimulation applications also promotes device durability. Nitinol-based conductor materials, such as NiTi-DFT-30%Ag wire 101 described in detail above, can perform at higher strain-loading without fracture as compared to conventional 35N LT-based systems by 50 to 100% based on lab bench results in coil and wire fatigue testing respectively, as demonstrated herein. The electrical resistivities of both nitinol (75-85 $\mu$ohm-cm) and 35N LT (103 $\mu$ohm-cm) based systems with and without silver core are comparable to allow similar form and fit into wire-, coil-, strand- and cable-based designs.

A materials system capable of nitinol shape programming even with an electrically insulative layer such as insulator 110 (Fig. 2) may also enable new lead solutions. High temperature insulative coatings 110 such as polyimide or PTFE may be used while enabling secondary or tertiary thermal shaping of leads to shapes that compliment anatomical placement. For example, an improved lead for left ventricular stimulation through the coronary sinus (CS) in cardiac resynchronization therapy (CRT) may be provided. Here, a CRT lead of polyimide-coated nitinol-silver, such as described above with respect to wire 105, can be heat programmed or shape set to a secondary helix or curve to compliment passive fixation in the CS enabling good passive anchoring and wall contact, in the manner of a nitinol-based self-expanding vascular stent. Such technology could prevent lead dislodgement and CS dissection, or provide more durable multifunctional leads by providing gentle but thorough wall apposition in a device with improved delivery navigation and deployment.

## Claims

1. A coiled or wound composite wire comprising:

   a shell made of a nickel-titanium alloy, and defining a hollow cavity having an inner diameter;
   a core made of a conductive metal material, received within the hollow cavity, and defining an outer diameter equal to the inner diameter; and
   a polyimide coating disposed over the outer diameter of the shell, the polyimide coating having a thermal degradation, as measured by thermogravimetric analysis, of less than 10% mass at 500 °C for 10 minutes or less in an inert environment,
   wherein the composite wire exhibits a fatigue endurance surviving at least $10^8$ cycles at 0.5% alternating strain.

2. The composite wire of claim 1, wherein: the shell and the core are made of medical-grade materials; and/or the core is centered in the hollow cavity; and/or the core consists of silver and incidental impurities; and/or the shell consists of about approximately 50 atomic % nickel, balance titanium and incidental impurities.

3. The composite wire of claim 1 or claim 2, wherein the composite wire exhibits at least twice the work energy to tensile fracture compared to the same wire configuration substituting CoNiCrMo for NiTi.

4. The composite wire of any preceding claim, wherein the core defines between 10% and 95% of an overall cross-sectional area of the shell and the core combined.

5. The composite wire of any preceding claim, wherein the composite wire is a drawn construct.

6. The composite wire of any preceding claim, wherein the composite wire is a shape set construct.

7. A medical device comprising a composite wire in accordance with any preceding claim.

8. A multifilament cable construct comprising:

   a plurality of wires, each of the wires comprising:

   a shell made of nickel-titanium alloy and defining a hollow cavity having an inner diameter; and
   a core made of a conductive metal material and received within the hollow cavity and defining an outer diameter equal to the inner diameter,
   wherein the plurality of wires are configured into a helical shape which holds the helical shape without external forcing; and

a polyimide coating surrounding the plurality of wires, the polyimide coating having a thermal degradation, as measured by thermogravimetric analysis, of less than 10% mass at 500 °C for 10 minutes or less in an inert environment.

9. The multifilament cable construct of claim 8, wherein the multifilament cable construct exhibits a fatigue endurance surviving at least $10^7$ cycles at 15% structural strain cycling.

10. The multifilament cable construct of claim 8, wherein the coiled or wound wire comprises a coiled monofilament wire exhibiting a fatigue endurance surviving at least $10^8$ cycles at 0.5% alternating strain.

11. The multifilament cable construct of any of claims 8-10, wherein the core consists of silver and incidental impurities.

12. The multifilament cable construct of any one of claims 8-11, wherein the shell consists of about approximately 50 atomic % nickel, balance titanium and incidental impurities.

13. A method of making a wire comprising:

inserting a conductive core into a hollow cavity of a shell made of nickel-titanium alloy to create a composite wire in which the core defines an outer diameter equal to an inner diameter of the hollow cavity;
coating the composite wire with a polyimide coating, and
after the step of coating the composite wire, shape setting the composite wire.

14. The method of claim 13, wherein the step of shape setting comprises:

constraining the composite wire to a desired shape; and
thermally processing the composite wire to retain the desired shape.

15. The method of claim 13, wherein the step of thermally processing comprises heating the composite wire and the coating to at least 400°C or at least 550°C.


**Patentansprüche**

1. Gewickelter oder aufgewickelter Verbunddraht, umfassend:

eine Hülle, hergestellt aus einer Nickel-Titan-Legierung, die einen Hohlraum mit einem Innendurchmesser definiert;
einen Kern, hergestellt aus einem leitfähigen Metallmaterial, der in dem Hohlraum aufgenommen ist, und einen Außendurchmesser definiert, der dem Innendurchmesser entspricht; und
eine Polyimidbeschichtung, die über dem Außendurchmesser der Hülle aufgebracht ist, wobei die Polyimidbeschichtung einen thermischen Abbau aufweist, wie er mittels thermogravimetrischer Analyse gemessen wird, von weniger als 10 Massenprozent bei 500 °C für 10 Minuten oder weniger in einer inerten Umgebung, wobei der Verbunddraht eine Ermüdungsfestigkeit aufweist, die mindestens $10^8$ Zyklen bei einer Wechselbeanspruchung von 0,5 % übersteht.

2. Verbunddraht nach Anspruch 1, wobei: die Hülle und der Kern aus Materialien in medizinischer Qualität bestehen; und/oder der Kern in dem Hohlraum zentriert ist; und/oder der Kern aus Silber und unvermeidbaren Verunreinigungen besteht; und/oder die Hülle aus etwa 50 Atom-% Nickel, Rest Titan und unvermeidbaren Verunreinigungen besteht.

3. Verbunddraht nach Anspruch 1 oder Anspruch 2, wobei der Verbunddraht im Vergleich zu der gleichen Drahtkonfiguration, bei der NiTi durch CoNiCrMo ersetzt ist, mindestens die doppelte Brucharbeit bei Zugbeanspruchung aufweist.

4. Verbunddraht nach einem vorstehenden Anspruch, wobei der Kern zwischen 10 % und 95 % einer Gesamtquerschnittsfläche der Hülle und des Kerns zusammen definiert.

5. Verbunddraht nach einem vorstehenden Anspruch, wobei der Verbunddraht ein gezogener Verbundkörper ist.

**6.** Verbunddraht nach einem vorstehenden Anspruch, wobei der Verbunddraht ein formeingestellter Verbundkörper ist.

**7.** Medizinische Vorrichtung, umfassend einen Verbunddraht nach einem vorstehenden Anspruch.

**8.** Mehrfilament-Kabelstruktur, umfassend:
eine Vielzahl von Drähten, wobei jeder der Drähte umfasst:

eine Hülle, hergestellt aus einer Nickel-Titan-Legierung, die einen Hohlraum mit einem Innendurchmesser definiert; und

einen Kern, hergestellt aus einem leitfähigen Metallmaterial, der in dem Hohlraum aufgenommen ist und einen Außendurchmesser definiert, der dem Innendurchmesser entspricht, wobei die Vielzahl von Drähten zu einer spiralförmigen Gestalt ausgebildet ist, die die spiralförmige Gestalt ohne äußere Krafteinwirkung hält; und

eine Polyimidbeschichtung, die die Vielzahl von Drähten umgibt, wobei die Polyimidbeschichtung einen thermischen Abbau aufweist, wie er mittels thermogravimetrischer Analyse gemessen wird, von weniger als 10 Massenprozent bei 500 °C für 10 Minuten oder weniger in einer inerten Umgebung.

**9.** Mehrfilament-Kabelstruktur nach Anspruch 8, wobei die Mehrfilament-Kabelstruktur eine Ermüdungsfestigkeit aufweist, die mindestens $10^7$ Zyklen bei einer strukturellen Dehnung mit zyklischer Beanspruchung von 15% übersteht.

**10.** Mehrfilament-Kabelstruktur nach Anspruch 8, wobei der gewickelte oder aufgewickelte Draht einen gewickelten Monofilamentdraht umfasst, der eine Ermüdungsfestigkeit aufweist, die mindestens $10^8$ Zyklen bei einer Wechselbeanspruchung von 0,5 % übersteht.

**11.** Mehrfilament-Kabelstruktur nach einem der Ansprüche 8-10, wobei der Kern aus Silber und unvermeidbaren Verunreinigungen besteht.

**12.** Mehrfilament-Kabelstruktur nach einem der Ansprüche 8-11, wobei die Hülle aus etwa 50 Atom-% Nickel, Rest Titan und unvermeidbaren Verunreinigungen besteht.

**13.** Verfahren zum Herstellen eines Drahtes, umfassend:

Einführen eines leitfähigen Kerns in einen Hohlraum einer Hülle, hergestellt aus einer Nickel-Titan-Legierung, um einen Verbunddraht herzustellen, in dem der Kern einen Außendurchmesser definiert, der dem Innendurchmesser des Hohlraums entspricht;
Beschichten des Verbunddrahtes mit einer Polyimidbeschichtung, und
nach dem Schritt des Beschichtens des Verbunddrahtes, Formgeben des Verbunddrahtes.

**14.** Verfahren nach Anspruch 13, wobei der Schritt des Formgebens umfasst:

Zwangsführen des Verbunddrahtes in eine gewünschte Form; und
thermisches Behandeln des Verbunddrahtes, um die gewünschte Form beizubehalten.

**15.** Verfahren nach Anspruch 13, wobei der Schritt der thermischen Behandlung ein Erhitzen des Verbunddrahts und der Beschichtung auf mindestens 400 °C oder mindestens 550 °C umfasst.

**Revendications**

**1.** Fil composite enroulé ou spiralé comprenant :

une coque en alliage nickel-titane et définissant une cavité creuse présentant un diamètre interne ;
un noyau constitué d'un matériau métallique conducteur, reçu dans la cavité creuse, et définissant un diamètre externe égal au diamètre interne ; et
un revêtement en polyimide disposé sur le diamètre externe de la coque, le revêtement en polyimide présentant une dégradation thermique, telle qu'elle a été mesurée par analyse thermogravimétrique, inférieure à 10 % en masse à 500 °C pendant 10 minutes ou moins dans un environnement inerte,
dans lequel le fil composite présente une résistance à la fatigue d'au moins $10^8$ cycles sous une déformation alternée de 0,5 %.

2. Fil composite selon la revendication 1, dans lequel : la coque et le noyau sont fabriqués à partir de matériaux de qualité médicale ; et/ou le noyau est centré dans la cavité creuse ; et/ou le noyau consiste en de l'argent et des impuretés accidentelles ; et/ou la coque consiste en environ 50 % atomiques de nickel, le reste étant constitué de titane et d'impuretés accessoires.

3. Fil composite selon la revendication 1 ou la revendication 2, dans lequel le fil composite présente au moins deux fois l'énergie de travail à la rupture par traction par rapport à la même configuration de fil substituant du CoNiCrMo pour du NiTi.

4. Fil composite selon une quelconque revendication précédente, dans lequel le noyau définit entre 10 % et 95 % d'une zone de section transversale globale de la coque et du noyau combinés.

5. Fil composite selon une quelconque revendication précédente, dans lequel le fil composite est une construction étirée.

6. Fil composite selon une quelconque revendication précédente, dans lequel le fil composite est une structure à forme fixée.

7. Dispositif médical comprenant un fil composite conformément à une quelconque revendication précédente.

8. Structure de câble multifilament comprenant :
une pluralité de fils, chacun des fils comprenant :

une coque en alliage nickel-titane et définissant une cavité creuse présentant un diamètre interne ; et
un noyau constitué d'un matériau métallique conducteur et reçu à l'intérieur de la cavité creuse et définissant un diamètre externe égal au diamètre interne, dans lequel la pluralité de fils est configurée selon une forme hélicoïdale qui conserve cette forme hélicoïdale sans application de force extérieure ; et
un revêtement en polyimide entourant la pluralité de fils, le revêtement en polyimide présentant une dégradation thermique, telle que mesurée par analyse thermogravimétrique, inférieure à 10 % en masse à 500 °C pendant 10 minutes ou moins dans un environnement inerte.

9. Structure de câble multifilament selon la revendication 8, dans laquelle la structure de câble multifilament présente une résistance à la fatigue d'au moins $10^7$ cycles sous un cyclage de déformation structurelle de 15 %.

10. Structure de câble multifilament selon la revendication 8, dans laquelle le fil enroulé ou spiralé comprend un fil monofilament enroulé présentant une résistance à la fatigue d'au moins $10^8$ cycles sous une déformation alternée de 0,5 %.

11. Structure de câble multifilament selon l'une quelconque des revendications 8 à 10, dans laquelle le noyau consiste en de l'argent et des impuretés accessoires.

12. Structure de câble multifilament selon l'une quelconque des revendications 8 à 11, dans laquelle la coque est constituée d'environ 50 % atomiques de nickel, de titane et d'impuretés accessoires.

13. Procédé de fabrication d'un fil comprenant :

l'insertion d'un noyau conducteur dans une cavité creuse d'une coque en alliage nickel-titane pour créer un fil composite dans lequel le noyau définit un diamètre externe égal à un diamètre interne de la cavité creuse ;
le revêtement du fil composite avec un revêtement en polyimide, et
après l'étape de revêtement du fil composite, la mise en forme du fil composite.

14. Procédé selon la revendication 13, dans lequel l'étape de mise en forme comprend :

la contrainte du fil composite dans une forme souhaitée ; et
le traitement thermique du fil composite pour conserver la forme souhaitée.

**15.** Procédé selon la revendication 13, dans lequel l'étape de traitement thermique comprend le chauffage du fil composite et du revêtement à au moins 400 °C ou à au moins 550 °C.

Fig. 1

Fig. 2

Fig. 3

Fig. 4

Fig. 5

EP 4 569 523 B1

Fig. 6

EP 4 569 523 B1

Fig. 7(a)

EP 4 569 523 B1

Fig. 7(b)

Fig. 7(c)

Fig. 8(a)

Fig. 8(b)

Fig. 8(c)

Fig. 9

EP 4 569 523 B1

Fig. 10

Fig. 11

Fig. 12

Fig. 13

Fig. 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8121687 B2
- WO 2018183862 A1
- US 7420124 B
- US 7501579 B
- US 7745732 B
- US 39509009
- WO 2019217350 A
- US 8840735 B

**Non-patent literature cited in the description**

- **JEREMY E. SCHAFFER**. Structure-Property Relationships in Conventional and Nanocrystalline NiTi Intermetallic Alloy Wire. *Journal of Materials Engineering and Performance*, 2009, vol. 18, 582-587